**Europäisches Patentamt**

(19) **European Patent Office**

**Office européen des brevets**

(11) Veröffentlichungsnummer: **0 045 282**
**B1**

(12) **EUROPÄISCHE PATENTSCHRIFT**

(45) Veröffentlichungstag der Patentschrift:
**29.05.85**

(51) Int. Cl.⁴: **A 61 K 7/027**, A 61 K 7/48

(21) Anmeldenummer: **81810303.8**

(22) Anmeldetag: **24.07.81**

(54) **Antiherpetisch wirksamer Lippenstift und dessen Verwendung zur Behandlung von Erkrankungen der Lippen und anderer Gesichtspartien, hervorgerufen durch menschliche Herpesviren.**

(30) Priorität: **30.07.80 CH 5810/80**
**02.10.80 CH 7361/80**

(43) Veröffentlichungstag der Anmeldung:
**03.02.82 Patentblatt 82/5**

(45) Bekanntmachung des Hinweises auf die Patenterteilung:
**29.05.85 Patentblatt 85/22**

(84) Benannte Vertragsstaaten:
**BE CH DE FR IT LI LU NL SE**

(56) Entgegenhaltungen:
**EP - A - 0 000 133**
**EP - A - 0 012 115**
**DE - A - 2 715 711**
**FR - A - 2 029 004**
**GB - A - 781 150**
**US - A - 3 590 123**

(73) Patentinhaber: **CIBA-GEIGY AG, Postfach,**
**CH-4002 Basel (CH)**

(72) Erfinder: **Lukas, Bohumir, Dr., Florastrasse 18/4,**
**CH-4057 Basel (CH)**
Erfinder: **Fischer, Franz Xaver, Dr., Säckingerstrasse 14,**
**CH-4058 Basel (CH)**
Erfinder: **Aeschlimann, Elfy, Haselrain 31,**
**CH-4125 Riehen (CH)**

## Beschreibung

Die Erfindung betrifft einen neuen, antiherpetisch wirksamen Lippenstift und dessen Verwendung zur Behandlung von Erkrankungen der Lippen und anderer Gesichtspartien, hervorgerufen durch menschliche Herpesviren.

Pharmazeutische Präparate zur topischen Behandlung von Virusinfektionen, insbesondere Infektionen durch Herpes-Viren, vor allem durch Herpesvirus hominis, die eine antiviral wirksame Kombination eines sulfatierten Polysaccharids oder sulfatierten Polymeren oder deren Salzen und Zinkionen in Form von dissoziierbaren Zinksalzen enthalten, sind aus der EU-OS 133 und der EU-OS 12 115 bekannt. Darin sind auch verschiedene an sich übliche topische Anwendungsformen wie Tinkturen, Lösungen, Cremes, Salben und Gele beschrieben.

Herpes labialis stellt eine besonders häufige und auch ästhetisch störende Herpes-Erkrankung dar. Als geeignete topische Anwendungsformen für die Behandlung von Herpes labialis könnten auch Lippenstifte in Betracht kommen, welche die obige Wirkstoffkombination enthalten, nachdem Lippenstifte immer häufiger auch in der Pflegekosmetik, d. h. zum Auftragen von Pflege- und Schutzstoffen, angewendet werden. Eigene Versuche haben jedoch gezeigt, daß die obenerwähnte Wirkstoffkombination in den bekannten Lippenstiftgrundlagen der Dekorations- und Pflegekosmetik nicht die z. B. aus der Anwendung in Form von Gelen bekannte starke Wirkung entfaltet, sondern erheblich schwächer wirkt, weil Bestandteile dieser Grundlagen, z. B. Wachse, höhere Fettalkohole und Fette an sich die Vesikelbildung und Infektiosität eher fördern und die Abheilung verzögern.

Es wurde nun überraschenderweise eine in ihrer Konsistenz und ihren weiteren Charakteristika zur Herstellung von Lippenstiften geeignete Grundlage gefunden, in der die Wirksamkeit der oben genannten Wirkstoffkombination voll erhalten ist. Der auf dieser Erkenntnis beruhende, erfindungsgemäße antiherpetische Lippenstift ist gekennzeichnet durch einen Gehalt an a) einer antiherpetisch wirksamen Kombination von mindestens einem sulfatierten Polysaccharid oder sulfatierten Polymeren oder mindestens einem Salz eines solchen und einem dissoziierbaren Zinksalz, und b) einem Polyäthylenglykolgemisch als Lippenstiftgrundlage, dessen Komponenten aus 15—30 Gewichtsprozent niedrigmolekularem, flüssigem Polyäthylenglykol mit einem mittleren Molekulargewicht zwischen 300 und 400 und 85—70 Gew.-% höhermolekularem, festem Polyäthylenglykol mit einem mittleren Molekulargewicht zwischen 1000 und 4000 bestehen.

Wie in den in der obengenannten Patentschrift beschriebenen Applikationsformen kann auch in den antiherpetischen Lippenstiften gemäß vorliegender Erfindung die synergistische Wirkung der beiden Wirkstoffkomponenten noch verstärkt werden, indem den beiden Wirkstoffkomponenten noch ein oder mehrere Polyoxyäthylensorbitan-fettsäureester, insbesondere Polyoxyäthylensorbitan-monostearat, -monolaurat und/oder -monooleat zugefügt werden. Ferner kann man auch weitere oberflächenaktive Stoffe, wie z. B. Sorbitan-monostearat, -monolaurat und/oder monooleat zufügen.

Unter sulfatierten Polysacchariden werden Polysaccharide verstanden, in denen einwertige Schwefelsäurereste $-SO_2-OH$ mit Sauerstoffatomen und/oder, falls, wie in Heparin vorhanden, Stickstoffatomen verbunden sind. Solche sulfatierten Polysaccharide können natürlicher Herkunft sein, wie Heparin, Chondroitinsulfat (Chondroitinschwefelsäure) oder Karrageenin, oder durch Sulfatierung von natürlichen oder partiell abgebauten Polysacchariden hergestellt sein, wie sulfatierte Amylopektine, sulfatierte Dextrane, sulfatierte Polyglucosen oder sulfatierte Polypentosen, vorzugsweise in der Form von geeigneten, pharmazeutisch annehmbaren Salzen, wie z. B. Kalium- und insbesondere Natriumsalzen.

Als solche seien das Natriumsalz von Heparin als übliche Handelsform des letzteren, weiter das Kalium-, das Lithium-, das Ammonium- und das Magnesiumsalz von Heparin, sowie das Natriumsalz von sulfatierten Dextranen genannt. Sulfatierte Polymere sind Sulfatierungsprodukte von Hydroxygruppen enthaltenden Polymeren, wie z. B. sulfatierte Polyvinylalkohole (Polyvinylsulfate) mit verschiedener durchschnittlicher Molekülgröße, die wiederum vorzugsweise in Form von pharmazeutisch annehmbaren Salzen, wie den Natrium- oder Kaliumsalzen, verwendet werden.

Als dissoziierbares Zinksalz kann z. B. Zinksulfat und dessen Hydrate, insbesondere das Heptahydrat, $ZnSO_4 \cdot 7 H_2O$, aber auch ein anderes dissoziierbares Zinksalz, wie z. B. Zinkchlorid, Zinkacetat oder Zinkcitrat, oder das Zinksalz einer Säure oder eines andern Stoffes von saurem Charakter und eigenen biologischen, z. B. antibakteriellen oder antiphlogistischen, Eigenschaften, wie z. B. Zink-sudoxicam (Zinksalz des 4-Hydroxy-2-methyl-N-(2-thiazolyl)-1,2-benzothiazin-3-carboxamid-1,1-dioxid) verwendet werden.

Die erfindungsgemäßen Lippenstiftgrundlagen lassen sich erzielen durch Kombination eines niedermolekularen, flüssigen Polyäthylenglykols (PEG) mit höhermolekularen festen PEG. Als niedermolekulare PEG kommen solche mit einem mittleren Molekulargewicht zwischen 300 und 400 in Frage, als höhermolekulare PEG sind solche mit einem mittleren Molekulargewicht zwischen 1000 und 4000 verwendbar. Insbesondere eignen sich Mischungen von PEG 400 und PEG 1000 mit einem geringen Zusatz von PEG 4000. Die Mengenanteile von flüssigem zu festem PEG liegen je nach verwendeten PEG-Typen zwischen 15 + 85 und 30 + 70 Teilen. Mit diesen PEG-Mischungen werden formbeständige Lippenstifte erhalten, die bei Gebrauch einen entsprechenden Anrieb auf den zu behandelnden Lippen oder Gesichtspartien gewährleisten.

Der erfindungsgemäße Lippenstift kann noch kleine Mengen von Polyäthylenglykolen mit einem Molekulargewicht über 4000, Glycerin sowie übliche Zusatzstoffe wie Farbstoffe, Aromastoffe, Riechstoffe und Lichtschutzmittel, enthalten. Ferner können die Geweberegeneration fördernde Stoffe (z. B. Allantoin) zugesetzt werden. Ein Zusatz von Konservierungsmitteln erübrigt sich, da der erfindungsgemäße Lippenstift selbstkonservierende Eigenschaften aufweist.

Die vorliegende Erfindung betrifft insbesondere Lippenstifte, die sulfatierte Polysaccharide, sulfatierte Polymere oder Salze von solchen, wie Heparin-Natrium, und Zinkionen in Form von dissoziierbaren Zinksalzen in einem Verhältnis von 1 mg zu 0,18 bis 4,5 mg, und gegebenenfalls Polyoxyäthylensorbitan-monosterat, -monolaurat und/oder -monooleat oder Sorbitan-monostearat, -monolaurat und/oder -monooleat enthalten. Für Heparin-Natrium beziehen sich obige Mengenangaben auf solches mit 160 USP-E/mg, von anderem Heparin-Natrium sind gleiche UPS-E-Mengen einzusetzen. Dem obigen Mengenbereich an Zinkionen entspricht z. B. für Zinksulfat-heptahydrat, $ZnSO_4 \cdot 7 H_2O$, als dissoziierbares Zinksalz ein Mengenbereich von 0,8—20 mg. Solche Lippenstifte enthalten beispielsweise pro g 0,2 bis 10 mg, insbesondere 1—5 mg eines sulfatierten Polysaccharids oder sulfatierten Polymeren, oder eines Salzes eines solchen, beispielsweise 32 bis 1600 USP-E, insbesondere 80 bis 800 USP-E Heparin-Natrium, und 0,18 bis 18 mg Zinkionen, entsprechend z. B. ca. 0,8 bis 80 mg $ZnSO_4 \cdot 7 H_2O$, und gegebenenfalls zusätzlich insgesamt 0,2 bis 50 mg von Polyoxyäthylensorbitan-monostearat, -monolaurat und/oder -monooleat oder Sorbitan-monostearat, -monolaurat und/oder -monooleat. Besonders bevorzugt ist ein Gehalt von 160—480 USP-E Heparin-Natrium, 0,45 bis 4,5 mg Zinkionen in Form von dissoziierbaren Zinksalzen und gegebenenfalls zusätzlich insgesamt 1,0 bis 20 mg von Polyoxyäthylensorbitan-monostearat, -monolaurat und/oder -monooleat oder Sorbitan-monostearat, -monolaurat und/oder -monooleat pro g oder ml.

Anstelle des Heparins oder dessen Salzen kann auch eine antiherpetisch wirkungsgleiche Menge eines andern sulfatierten Polysaccharids oder eines sulfatierten Polymeren oder eines ihrer Salze verwendet werden.

Der erfindungsgemäße Lippenstift wird zur Behandlung des Herpes der Lippen und anderer Gesichtspartien so frühzeitig wie möglich mehrmals täglich bis zum Abklingen der Symptome bzw. bis zur Abheilung auf die erkrankten Lippen aufgetragen.

Die nachfolgenden Beispiele beschreiben die Herstellung eines Lippenstiftes; sie sollen jedoch den Umfang der Erfindung in keiner Weise beschränken.

Beispiel 1

Zur Herstellung von 10 kg Lippenstiftmasse werden 7,73 kg Polyäthylenglykol 1000 und 190 g Polyäthylenglykol 4000 mit 1,4 kg Polyäthylenglykol 400, 160 g Polyoxyäthylensorbitan-monostearat (TWEEN 60) und 40 g Polyoxyäthylensorbitan-monostearat (TWEEN 80) zusammengeschmolzen. Hierauf werden 80 g feinstgemahlenes Heparin-Natrium (mit einer biologischen Aktivität von 160 USP-E/mg) mit 400 g feinstgemahlenem Zinksulfat-heptahydrat ($ZnSO_4 \cdot 7 H_2O$) vermischt. Diese Pulvermischung wird sodann mittels kräftigem Rühren in der Grundlagenschmelze verteilt. Das Vergießen in Lippenstift-Formen erfolgt bei einer knapp oberhalb dem Erstarrungspunkt liegenden Temperatur.

TWEEN 60 und TWEEN 80 sind geschützte Markenbezeichnungen der ICI of America Inc., Stamford, Connecticut (USA).

Beispiel 2

Herstellung von Lippenstiften analog zu Beispiel 1, jedoch unter Verwendung folgender Wirk- und Hilfsstoffmengen: 6,76 kg Polyäthylenglykol 3000, 2,90 kg Polyäthylenglykol 300, 80 g Polyoxyäthylensorbitan-monostearat (TWEEN 60), 20 g Polyoxyäthylensorbitan-monooleat (TWEEN 80), 40 g feinstgemahlenes Heparin-Natrium und 200 g feinstgemahlenes Zinksulfat-heptahydrat.

Beispiel 3

Herstellung von Lippenstiften analog zu Beispiel 1, jedoch unter Verwendung folgender Wirk- und Hilfsstoffmengen: 6,60 kg Polyäthylenglykol 3000, 2,91 kg Polyäthylenglykol 300, 200 g Glycerin 98%, 40 g Polyoxyäthylensorbitan-monostearat (TWEEN 60), 10 g Polyoxyäthylensorbitan-monooleat (TWEEN 80), 40 g feinstgemahlenes Heparin-Natrium und 200 g feinstgemahlenes Zinksulfat-heptahydrat.

Beispiel 4

Herstellung von Lippenstiften analog zu Beispiel 1, jedoch unter Verwendung folgender Wirk- und Hilfsstoffmengen: 6,88 kg Polyäthylenglykol 3000, 2,95 kg Polyäthylenglykol 400, 40 g Polyoxyäthylensorbitan-monostearat (TWEEN 60), 10 g Polyoxyäthylensorbitan-monooleat (TWEEN 80), 20 g feinstgemahlenes Heparin-Natrium und 100 g feinstgemahlenes Zinksulfat-heptahydrat.

Beispiel 5

Herstellung von Lippenstiften analog zu Beispiel 1, jedoch unter Verwendung folgender Wirk- und Hilfsstoffmengen: 7,553 kg Polyäthylenglykol 1000, 246 g Polyäthylenglykol 4000, 1,946 kg Polyäthylenglykol 400, 60 g Polyoxyäthylensorbitan-monostearat (TWEEN 60), 15 g Polyoxyäthylensorbitan-monooleat (TWEEN 80), 30 g feinstgemahlenes Heparin-Natrium und 150 g feinstgemahlenes Zinksulfat-heptahydrat.

### Beispiel 6

Herstellung von Lippenstiften analog zu Beispiel 1, jedoch unter Verwendung folgender Wirk- und Hilfsstoffmengen: 7,600 kg Polyäthylenglykol 1000, 248 g Polyäthylenglykol 4000, 1,957 kg Polyäthylenglykol 400, 60 g Polyoxyäthylensorbitan-monostearat (TWEEN 60), 15 g Polyoxyäthylensorbitan-monooleat (TWEEN 80), 30 g feinstgemahlenes Heparin-Natrium und 90 g feinstgemahlenes Zinksulfat-heptahydrat.

### Beispiel 7

Herstellung von Lippenstiften analog zu Beispiel 1, jedoch unter Verwendung folgender Wirk- und Hilfsstoffmengen: 7,80 kg Polyäthylenglykol 1000, 200 g Polyäthylenglykol 4000, 1,520 kg Polyäthylenglykol 400, 200 g Glycerin 98%, 80 g Polyoxyäthylensorbitan-monostearat (TWEEN 60), 20 g Polyoxyäthylensorbitan-monooleat (TWEEN 80), 30 g feinstgemahlenes Heparin-Natrium und 150 g feinstgemahlenes Zinksulfat-heptahydrat.

### Beispiel 8

Analog Beispiel 7 jedoch unter Verwendung von Sorbitanmonostearat (SPAN 60) anstelle von Polyoxyäthylensorbitan-monostearat und Sorbitan-monooleat (SPAN 80) anstelle von Polyoxyäthylensorbitan-monooleat.

**Patentansprüche**

1. Antiherpetisch wirksamer Lippenstift, gekennzeichnet durch einen Gehalt an a) einer antiherpetisch wirksamen Kombination von mindestens einem sulfatierten Polysaccharid oder sulfatierten Polymeren oder mindestens einem Salz eines solchen und einem dissoziierbaren Zinksalz, und b) einem Polyäthylenglykolgemisch als Lippenstiftgrundlage, dessen Komponenten aus 15—30 Gewichtsprozent niedrigmolekularem, flüssigem Polyäthylenglykol mit einem mittleren Molekulargewicht zwischen 300 und 400 und 85—70 Gew.-% höhermolekularem, festem Polyäthylenglykol mit einem mittleren Molekulargewicht zwischen 1000 und 4000 bestehen.

2. Antiherpetisch wirksamer Lippenstift gemäß Anspruch 1, gekennzeichnet durch einen zusätzlichen Gehalt an Polyoxyäthylensorbitan-monostearat, -monolaurat und/oder -monooleat oder Sorbitan-monostearat, -monolaurat und/oder -monooleat.

3. Antiherpetisch wirksamer Lippenstift gemäß Anspruch 1, gekennzeichnet durch einen Gehalt an sulfatierten Polysacchariden, sulfatierten Polymeren oder Salzen von solchen und Zinkionen in Form von dissoziierbaren Zinksalzen in einem Mengenverhältnis von 1 mg zu 0,18 bis 18 mg.

4. Antiherpetisch wirksamer Lippenstift gemäß Anspruch 3, gekennzeichnet durch einen Gehalt an sulfatierten Polysacchariden, sulfatierten Polymeren oder Salzen von solchen und Zinkionen in Form von dissoziierbaren Zinksalzen in einem Mengenverhältnis von 1 mg zu 0,18 bis 4,5 mg.

5. Antiherpetisch wirksamer Lippenstift gemäß Anspruch 3, gekennzeichnet durch einen Gehalt an Heparin-Natrium und Zinkionen in Form von dissoziierbaren Zinksalzen in einem Verhältnis von 160 USP-E zu 0,18 bis 18 mg.

6. Antiherpetisch wirksamer Lippenstift gemäß Anspruch 3, gekennzeichnet durch einen Gehalt, pro g, von 0,2 bis 10 mg eines sulfatierten Polysaccharids, sulfatierten Polymeren oder Salzes eines solchen und 0,18 bis 18 mg Zinkionen in Form von dissoziierbaren Zinksalzen.

7. Antiherpetisch wirksamer Lippenstift gemäß Anspruch 3, gekennzeichnet durch einen Gehalt, pro g, von 1 bis 5 mg eines sulfatierten Polysacchrids, sulfatierten Polymeren oder Salzes eines solchen und 0,18 bis 18 mg Zinkionen in Form von dissoziierbaren Zinksalzen.

8. Antiherpetisch wirksamer Lippenstift gemäß Anspruch 3, gekennzeichnet durch einen Gehalt, pro g oder ml, von 32 bis 1600 USP-E Heparin oder einem pharmazeutisch annehmbaren Salz davon und 0,18 bis 18 mg Zinkionen in Form von dissoziierbaren Zinksalzen.

9. Antiherpetisch wirksamer Lippenstift gemäß Anspruch 3, gekennzeichnet, durch einen Gehalt, pro g, von 80 bis 800 USP-E Heparin oder einem pharmazeutisch annehmbaren Salz davon und 0,18 bis 18 mg Zinkionen in Form von dissoziierbaren Zinksalzen.

10. Antiherpetisch wirksamer Lippenstift gemäß Anspruch 3, gekennzeichnet, durch einen Gehalt, pro g, von 160 bis 480 USP-E Heparin oder einem pharmazeutisch annehmbaren Salz davon und 0,45 bis 4,5 mg Zinkionen in Form von dissoziierbaren Zinksalzen.

11. Antiherpetisch wirksamer Lippenstift gemäß Ansprüchen 3—10, dem die Zinkionen in Form von $ZnSO_4 \cdot 7 H_2O$ als dissoziierbarem Zinksalz zugefügt ist.

12. Antiherpetisch wirksamer Lippenstift gemäß Ansprüchen 6—10, gekennzeichnet durch einen zusätzlichen Gehalt, pro g, von insgesamt 0,2 bis 50 mg von Polyoxyäthylensorbitan-monostearat, -monolaurat und/oder -monooleat oder von Sorbit-monostearat, -monolaurat und/oder -monooleat.

13. Antiherpetisch wirksamer Lippenstift gemäß Ansprüchen 6—10, gekennzeichnet durch einen zusätzlichen Gehalt, pro g, von insgesamt 1,0 bis 20 mg von Polyoxyäthylensorbitan-monostearat, -monolaurat und/oder -monooleat oder Sorbitan-monostearat, -monolaurat und/oder -monooleat.

14. Antiherpetisch wirksamer Lippenstift gemäß einen der Ansprüche 1—4 und 6—13, dadurch gekennzeichnet, daß er ein pharmazeutisch annehmbares Salz eines sulfatierten Polysaccharids oder sulfatierten Polymers enthält.

15. Antiherpetisch wirksamer Lippenstift gemäß Anspruch 1, dadurch gekennzeichnet, daß er, als niedrigmolekulares Polyäthylenglykol, Polyäthylenglykol 400 und, als höhermolekulare Polyäthylenglykole, Polyäthylenglykol 1000 mit geringen Mengen PEG 4000 enthält.

## Claims

1. An antiherpetic lipstick which contains a) an antiherpetically effective combination comprising at least one sulphatised polysaccharide or sulphatised polymer or at least one salt thereof and a dissociable zinc salt and b) a polyethylene glycol mixture as lipstick base, the components of which consist of 15—30% by weight of a low molecular liquid polyethylene glycol having an average molecular weight from 300 to 400 and 85—70% by weight of a high molecular solid polyethylene glycol having an average molecular weight from 1000 to 4000.

2. An antiherpetic lipstick according to claim 1, which additionally contains polyoxyethylene sorbitan monostearate, monolaurate an/or monooleate or sorbitan monostearate, monolaurate and/or monooleate.

3. An antiherpetic lipstick according to claim 1, which contains sulphatised polysaccharides or sulphatised polymers or salts thereof and zinc ions in the form of dissociable zinc salts in a ratio of 1 mg : 0.18 to 18 mg.

4. An antiherpetic lipstick according to claim 3, which contains sulphatised polysaccharides or sulphatised polymers of salts thereof and zinc ions in the form of dissociable zinc salts in a ratio of 1 mg : 0.18 to 4.5 mg.

5. An antiherpetic lipstick according to claim 3, which contains heparin sodium and zinc ions in the form of dissociable zinc salts in a ratio of 160 USP units : 0.18 to 18 mg.

6. An antiherpetic lipstick according to claim 3, which contains, per g, 0.2 to 10 mg of a sulphatised polysaccharide or a sulphatised polymer of a salt thereof and 0.18 to 18 mg of zinc ions in the form of dissociable zinc salts.

7. An antiherpetic lipstick according to claim 3, which contains, per g, 1 to 5 mg of a sulphatised polysaccharide or a sulphatised polymer or a salt thereof and 0.18 to 18 mg of zinc ions in the form of dissociable zinc salts.

8. An antiherpetic lipstick according to claim 3, which contains, per g or ml, 32 to 1600 USP units of heparin or a pharmaceutically acceptable salt thereof and 0.18 to 18 mg of zinc ions in the form of dissociable zinc salts.

9. An antiherpetic lipstick according to claim 3, which contains, per g, 80 to 800 USP units of heparin or a pharmaceutically acceptable salt thereof and 0.18 to 18 mg of zinc ions in the form of dissociable zinc salts.

10. An antiherpetic lipstick according to claim 3, which contains, per g,160 to 480 USP units of heparin or a pharmaceutically acceptable salt thereof and 0.45 to 4.5 mg of zinc ions in the form of dissociable zinc salts.

11. An antiherpetic lipstick according to any one of claims 3 to 10, to which zinc ions are added in the form of $ZnSO_4 \cdot 7 H_2$ as dissociable zinc salt.

12. An antiherpetic lipstick according to any one of claims 6 to 10, which additionally contains per g, a total of 0.2 to 50 mg of polyoxyethylene sorbitan monostearate, monolaurate and/or monooleate or sorbitan monostearate, monolaurate and/or monooleate.

13. An antiherpetic lipstick according to claims 6 to 10, which additionally contains, per g, a total of 1.0 to 20 mg of polyoxyethylene sorbitan monostearate, monolaurate and/or monooleate or sorbitan monostearate, monolaurate and/or monooleate.

14. An antiherpetic lipstick according to any one of claims 1 to 4 and 6 to 13, which contains a pharmaceutically acceptable salt of a sulphatised polysaccharide or sulphatised polymer.

15. An antiherpetic lipstick according to claim 1, which contains, as low molecular polyethylene glycol, polyethylene glycol 400, and as high molecular polyethylene glycol, polyehtylene glycol 1000, together with small quantities of PEG 4000.

## Revendications

1. Bâton à lèvres possédant une activité anti-herpétique, caractérisé en ce qu'il contient a) une combinaison anti-herpétique active d'au moins un polysaccharide sulfaté ou polymère sulfaté ou d'au moins un sel d'un tel polysaccaride ou polymère et d'un sel de zinc dissociable, et b) un mélange de polyéthylène-glycols en tant que base du bâton à lèvres, dont les composants consistent en 15 à 30% en poids d'un polyéthylène-glycol liquide à bas poids moléculaire, de poids moléculaire moyen 300 à 400, et 85 à 70% en poids d'un polyéthylène-glycol solide à haut poids moléculaire, de poids moléculaire moyen 1.000 à 4.000.

2. Bâton à lèvres à activité anti-herpétique selon la revendication 1, caractérisé en ce qu'il contient en outre du monostéarate, du monolaurate et/ou du monooléate de sorbitanne polyoxyéthyléné ou du monostéarate, du monolaurate et/ou du monooléate de sorbitanne.

3. Bâton à lèvres possédant une activité anti-herpétique selon la revendication 1, caractérisé en ce qu'il contient des polysaccharides sulfatés, des polymères sulfatés ou des sels de ceux-ci et des ions zinc sous la forme de sels de zinc dissociables, dans des proportions relatives de 1 mg pour 0,18 à 18 mg.

4. Bâton à lèvres possédant une activité anti-herpétique selon la revendication 3, caractérisé en ce qu'il contient des polysaccharides sulfatés, des polymères sulfatés ou des sels de sulfite et des ions zinc sous la forme de sels de zinc dissociables dans des proportions relatives de 1 mg pour 0,18 à 4,5 mg.

5. Bâton à lèvres possédant une activité anti-herpétique selon la revendication 3, caractérisé en ce qu'il contient de l'héparine-sodium et des ions zinc sous la forme de sels de zinc dissociables dans des proportions relatives de 160 U-USP pour 0,18 à 18 mg.

6. Bâton à lèvres possédant une activité anti-herpétique selon la revendication 3, caractérisé en ce qu'il contient, pour 1 g, 0,2 à 10 mg d'un polysaccharide sulfaté, d'un polymère sulfaté ou d'un sel d'un tel polysaccharide ou polymère, et 0,18 à 18 mg d'ions zinc sous la forme de sels de zinc dissociables.

7. Bâton à lèvres possédant une activité anti-herpétique selon la revendication 3, caractérisé en ce qu'il contient, pour 1 g, de 1 à 5 mg d'un polysaccharide sulfaté, d'un polymère sulfaté ou d'un sel d'un tel polysaccharide ou polymère, et 0,18 à 18 mg d'ions zinc sous la forme de sels de zinc dissociables.

8. Bâton à lèvres possédant une activité anti-herpétique selon la revendication 3, caractérisé en ce qu'il contient, pour 1 g ou 1 ml, 32 à 1.600 U-USP d'héparine ou d'un sel de l'héparine acceptable pour l'usage pharmaceutique et de 0,18 à 18 mg d'ions zinc sous la forme de sels de zinc dissociables.

9. Bâton à lèvres possédant une activité anti-herpétique selon la revendication 3, caractérisé en ce qu'il contient, pour 1 g, de 80 à 800 U-USP d'héparine ou d'un sel de l'héparine acceptable pour l'usage pharmaceutique et de 0,18 à 18 mg d'ions zinc sous la forme de sels de zinc dissociables.

10. Bâton à lèvres possédant une activité anti-herpétique selon la revendication 3, caractérisé en ce qu'il contient, pour 1 g, de 160 à 480 U-ISP d'héparine ou d'un sel de l'héparine acceptable pour l'usage pharmaceutique et de 0,45 à 4,5 mg d'ions zinc sous la forme de sels de zinc dissociables.

11. Bâton à lèvres possédant une activité anti-herpétique selon les revendications 3 à 10, dans lequel on a introduit les ions zinc sous la forme de $ZnSO_4, 7 H_2O$ en tant que sel de zinc dissociable.

12. Bâton à lèvres possédant une activité anti-herpétique selon les revendications 6 à 10, caractérisé en ce qu'il contient en outre, pour 1 g, au total 0,2 à 50 mg de monostéarate, de monolaurate ou de monooléate de sorbitanne polyoxyéthyléné ou de monostéarate, de monolaurate et/ou de monooléate de sorbitanne.

13. Bâton à lèvres possédant une activité anti-herpétique selon les revendications 6 à 10, caractérisé en ce qu'il contient en outre, pour 1 g, au total 1,0 à 20 mg de monostéarate, monolaurate et/ou monooléate de sorbitanne polyoxyéthyléné ou de mono-stéarate, de monolaurate et/ou de monooléate de sorbitanne.

14. Bâton à lèvres possédant une activité anti-herpétique selon l'une des revendications 1 à 4 et 6 à 13, caractérisé en ce qu'il contient un sel acceptable pour l'usage pharmaceutique d'un polysaccharide sulfaté ou d'un polymère sulfaté.

15. Bâton à lèvres possédant une activité anti-herpétique selon la revendication 1, caractérisé en ce qu'il contient en tant que polyéthylène-glycol à bas poids moléculaire du polyéthylène-glycol 400 et en tant que polyéthylène-glycols à haut poids moléculaire, du polyéthylène-glycol 1.000 accompagné de petites quantités de polyéthylène-glycol 4.000.